# EUROPEAN PATENT APPLICATION

(11) **EP 1 138 669 A1**
(43) Date of publication of application: **04.10.2001**
(21) Application number: 99972201.0
(22) Date of filing: 12.11.1999
(51) Int. Cl.: C07C 309/59, C07C 309/60, C08L 65/00, C08L 59/00, C08L 101/00, C08K 5/42, H01G 9/02

(54) **BENZENESULFONIC ACID DERIVATIVE COMPOUNDS, PROCESS FOR PRODUCING THE SAME, AND USE THEREOF**

(30) Priority: 13.11.1998 JP 32327998; 13.11.1998 JP 32337098
(71) Applicant: Mitsui Chemicals, Inc., Tokyo 100-6070 (JP)
(72) Inventor: KUWAHARA, Masahiro, Mitsui Chemicals Inc., Yokohama-shi, Kanagawa 247-0006 (JP); NISHIYAMA, Shinichi, Mitsui Chemicals Inc., Sodegaura-shi, Chiba 299-0265 (JP)
(74) Representative: Cresswell, Thomas Anthony
(86) International application number: JP9906328
(87) International publication number: WO0029374

(57) **Abstract**

A benzosulfonic acid derivative compound which is utilized as a dopant for electron-conjugated polymer and are expressed by the following formula. 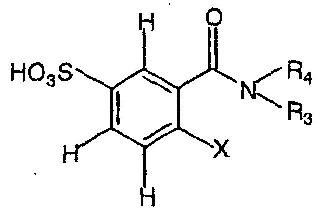 Wherein X is hydrogen or hydroxyl group, and R₃ and R₄ each are hydrogen, alkyl group, etc. Examples of the compound include 3-sulfobenzamide, 3-sulfobenzoylurea, N-methyl-3-sulfobenzamide, and 5-sulfosalicylamide. Doping an electron-conjugated polymer with the compound gives a conducting polymeric material which has high conductivity and excellent heat resistance and is usable as a conducting material suited to a cathode in a solid electrolytic capacitor.

## Description

### Technical Field of the Invention:

The invention relates to a novel benzenesulfonic acid derivative compound and a process for producing the same. The invention also relates to a dopant comprising the benzenesulfonic acid derivative compound which are applicable to electron-conjugated polymers, a conducting polymeric material obtained by a method like doping, and a solid electrolytic capacitor wherein the conducting polymeric material is used as a cathode material.

### Background of the Invention:

In recent years with the advance in electronics technology, novel electronic materials have been developed. In particular, remarkable technological innovation has been achieved in the area involving high performance organic materials. Even if the scope of view is restricted to the conductive materials alone, there have been developed conducting polymeric materials produced by way of doping electron-conjugated polymers such as polyacetylene, polyparaphenylene, polypyrrole, and polyaniline with some electron acceptive compounds as the dopant. And, there are even cases in which newly developed materials have been actually industrialized as the capacitor electrode material, battery electrode material, and antistatic material.

These electron-conjugated polymers exhibit an electric conductivity owing to changes of their transition to the oxidized state as they are doped with some electron acceptive compounds. It is due to such feature that said electron-conjugated polymers lose their electric conductivity in consequence of decomposition or chemical structural change which may be readily caused by heat or light energy, etc. With regard to the mechanism of such decomposition or chemical structural change, there have been suggested and explained the presence of a mechanism whereby the conjugated system is modified due to radical decomposition of molecules, a mechanism whereby the conjugated system is destroyed in consequence of some ionic decomposition, etc. Several countermeasures have been contemplated to cope with those problems.

For example, according to Japanese Laid-open Patent Publication No. 21281/1993 or Japanese Laid-open Patent Publication No. 21283/1993, suggestions are made to the effect that heat resistance of certain conducting polymeric materials can be enhanced by means of admixing phenol derivatives or phenoxide derivatives.

Also, according to Japanese Laid-open Patent Publication No. 286902/1997, there is indicated a material possessing the effects unique to both dopant and radical scavenger at the same time can be obtained by employing benzophenonesulfonic acid as the dopant.

Furthermore, according to Japanese Laid-open Patent Publication No. 25417/1997, there is suggested a conducting polymeric material that is doped with an aromatic sulfonic acid compound having at least one carboxyl group or hydroxyl group. According to this suggestion, an explanation is made to the effect that the proton donating effect can be attained in addition to the radical scanvenging effect by way of utilizing a benzoic acid derivative or a phenol derivative as the dopant, and thus is improved heat resistance of the conducting polymeric material.

These attempts are all aimed at establishing methods for preventing decomposition or structural change of the electron-conjugated polymer per se by way of modifying the molecule of the dopant. Nevertheless, if the molecule of the dopant thoroughly undergoes thermal decomposition or structural changes, heat resistance of the electron-conjugated polymer is also impaired.

Moreover, in consequence of the addition of the thermal decomposition preventing effect or the structural change preventing effect to the molecule of the dopant, the conductivity rendering effect, which is the inherent role of the molecule of a dopant, sometimes diminishes. For instance, according to Japanese Laid-open Patent Publication No. 286902/1997, polypyrrole doped with benzophenonesulfonic acid gives an electric conductivity of about 10 S/cm. Furthermore, according to Chem. Letter, 1996, 253 (1996) (K. Yamamoto, et al.), polypyrrole doped with 5-sulfosalicylic acid exhibited an electric conductivity of approx. 75 S/cm at best.

Moreover, electric properties of the complex five-membered ring polymer are apt to be influenced by the presence of traces of impurities. Therefore, it is important to remove impurities originated from the dopant as much as possible. It is for this reason that studies are required retrogressively as far back as the synthesis and purification stages of the dopant. The generally known method for synthesizing aromatic sulfonic acid compound is to isolate and purify its alkali metal salt or alkaline-earth metal salt. This method, however, gives rise to pronounced declines in electric properties and thermal properties of the electron-conjugated polymer such as polypyrrole due to residual alkali metal ions or alkaline-earth metal ions.

Meanwhile, there have been developed such solid electrolytic capacitors that comprise an anode constructed of a metal like tantalum, aluminum, etc. on whose surface is formed a film of oxide of tantalum, aluminum, etc., and a cathode constructed of manganese dioxide or tetracyano-quinodimethane (TCNQ) complex salt. Nevertheless, some comments have been made to the effect that in case where a solid electrolytic capacitor having a manganese dioxide cathode is used, the impedance increases throughout the high-frequency range due to the low dielectric constant of manganese dioxide, and in case of using TCNQ heat resistance is apt to decline due to the fact that TCNQ complex salt readily gets thermally decomposed.

Meanwhile, there have been discovered certain characteristics of the electron-conjugated polymer having conductivity such as the aforesaid polypyrrole and polyaniline. That is to say, such matters were found to demonstrate higher dielectric constant than those of manganese dioxide and superior heat resistance to TCNQ complex salt when they are utilized for the cathode.

It is on account of the interest focused on said characteristics that a number of improvements have been proposed concerning solid electrolytic capacitors wherein those conducting polymeric material are utilized for the cathode. For instance, Japanese Laid-open Patent Publication No. 37114/1985 discloses a solid electrolytic capacitor utilizing a complex five-membered ring polymer doped with a dopant as the solid electrolyte. Also, in Japanese Laid-open Patent Publication No. 29159/1994 there appears a description of a solid electrolytic capacitor comprising polyaniline as the solid electrolyte.

### Description of the Invention:

It is an object of the invention to provide a novel compound suited as a doping material capable of enhancing heat resistance without lowering, or virtually impairing an inherent electric conductivity of an electron-conjugated polymer, a process for producing the same, and such a dopant.

It is another object of the invention to provide a conducting polymeric material suited for a conductive material for a cathode in a solid electrolytic capacitor having good impedance characteristics even throughout a high-frequency range and also excellent heat resistance, and solid electrolytic capacitors wherein the conducting polymeric material is used.

Namely, the invention relates to a benzenesulfonic acid derivative compound expressed by a formula [1]. 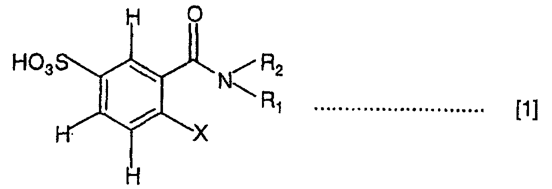 Wherein X is a hydrogen or a hydroxyl group; and
R₁ and R₂ are a hydrogen or any groups selected from the group of an alkyl group, an alkoxyl group, an aryl group, and an aminocarbonyl group, provided that they may be the same or different except that R₁ and R₂ are a hydrogen; and in case either one of R₁ and R₂ is a hydrogen, the other is a group selected from the group of an alkyl group, an alkoxyl group, an aryl group and an aminocarbonyl group.

Secondly, the invention relates to a process for producing a benzenesulfonic acid derivative compound expressed by a formula [3] by way of reacting 100 parts by weight of a benzamide compound expressed by a formula [2] with 500 parts by weight or less of a fuming sulfuric acid. 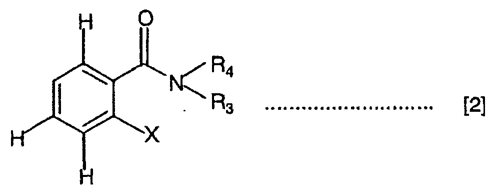 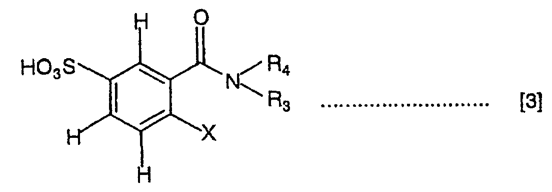 Wherein X is a hydrogen or a hydroxyl group; and
R₃ and R₄ are a hydrogen or any groups selected from the group of an alkyl group, an alkoxyl group, an aryl group, and an aminocarbonyl group, and may be the same or different.

In executing the producing process, it is preferable that a particular method be adopted so as to cause the benzamide compound to react with the fuming sulfuric acid, and after adjusting a sulfuric acid concentration of the reactant solution to 50 % by weight or more, deposit a sulfonate expressed by the formula [3] with acid in the reactant solution. It is furthermore desirable that a step be added so that the deposit from the aforesaid reactant solution is washed with an organic solvent.

Thirdly, the invention relates to a dopant comprising a benzenesulfonic acid derivative compound expressed by a formula [4]. 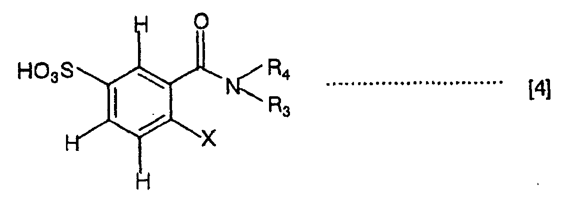 Wherein X, R₃, and R₄ are the same as expressed by the formula [3], respectively.

As preferable dopants expressed by the formula, there can be exemplified 3-sulfobenzamide, N-methyl-3-sulfobenzamide, N,N-dimethyl-3-sulfobenzamide, N-phenyl-3-sulfobenzamide, N,N-diphenyl-3-sulfobenzamide, 3-sulfobenzoylurea, 5-sulfosalicylamide, N-methyl-5-sulfosalicylamide, N,N-dimethyl-5-sulfosalicylamide, N-phenyl-5-sulfosalicylamide, etc.

Fourthly, the invention relates to a conducting polymeric material comprising the benzenesulfonic acid derivative compound expressed by the formula [4] which is included in a polymer having the electron-conjugated molecular structure.

What is preferred as the polymer is a π-electron-conjugated polymeric compound having at least one kind of repeating unit expressed by a formula [5], [6], or [7]. Particularly preferred is polypyrrole. 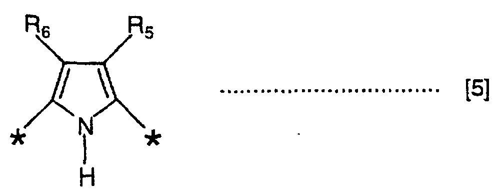 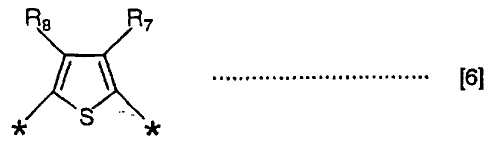 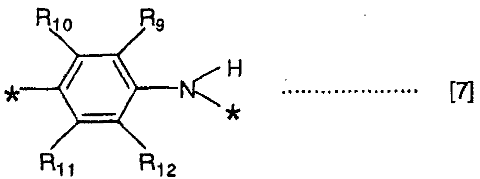 Wherein R₅ - R₁₂ may be the same or different, and are each either one of a hydrogen, an alkyl group, and an alkoxyl group. The symbol " 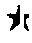 " denotes the position where the repeating unit is bonded.

### Brief Description of the Drawing:

Fig. 1 is an NMR chart on N-methyl-3-sulfobenzamide.
Fig. 2 is an NMR chart on N,N-dimethyl-3-sulfobenzamide.
Fig. 3 is an NMR chart on 3-sulfobenzoylurea.

### Description of the Preferred Embodiments:

The invention and its composing elements are explained in detail as follows.

### Benzenesulfonic Acid Derivative compound

A benzenesulfonic acid derivative compound of the invention is a compound expressed by a following formula [1]. 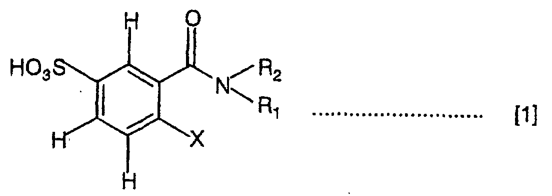 Wherein X is a hydrogen or a hydroxyl group; and
R₁ and R₂ are a hydrogen or any groups selected from the group of an alkyl group, an alkoxyl group, an aryl group, and an aminocarbonyl group (-CONH₂), provided that they may be the same or different except that R₁ and R₂ are a hydrogen; and in case either one of R₁ and R₂ is a hydrogen, the other is a group selected from the group of an alkyl group, an alkoxyl group, an aryl group and an aminocarbonyl group.

It is preferable that the alkyl group, the alkoxyl group, and the aryl group are either one of linear, branched or cyclic groups having 1 - 20 carbon atoms. There can be cited as their examples such groups as methyl, ethyl, propyl, butyl, octyl, methoxy, ethoxy, phenyl, methoxyphenyl, ethoxyethyl, methoxyethyl, cyclohexyl, etc. It is especially preferable that R₁ and R₂ are either ones of hydrogen, methyl group, phenyl group, and aminocarbonyl group.

As specific compounds expressed by the formula [1] wherein X is hydrogen there can be exemplified such 3-sulfobenzamides as N-methyl-3-sulfobenzamide, N,N-dimethyl-3-sulfobenzamide, N-phenyl-3-sulfobenzamide, N,N-diphenyl-3-sulfobenzamide, 3-sulfobenzoylurea.

Moreover, there can be cited as specific compounds expressed by the formula [1] wherein X is hydroxyl group such 5-sulfosalicylamides as N-methyl-5-sulfosalicylamide, N,N-dimethyl-5-sulfosalicylamide, N-phenyl-5-sulfosalicylamide.

The benzenesulfonic acid derivative compound having such chemical structure may be utilized as a raw material suited to synthesis of various organic compounds, besides being utilizable as a dopant as mentioned later.

### Process for Producing Benzenesulfonic Acid Derivative compound

The benzenesulfonic acid derivative compound can be produced via such synthesizing routes as sulfonation of a benzamide compounds or amidation of 3-sulfobenzoic acid. In particular, 3-sulfobenzamide expressed by a formula [3] can be suitably manufactured via a sulfonation process which is executed by bringing a benzamide compound including salicylamide expressed by a formula [2] into contact with an adequate sulfonating agent such as sulfuric acid, fuming sulfuric acid, sulfur trioxide, sulfur trioxide-pyridine complex, amidosulfonic acid.

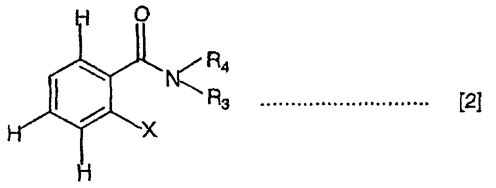

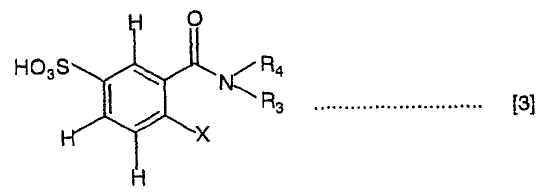

Wherein X is a hydrogen or a hydroxyl group; and
R₃ and R₄ are a hydrogen or any groups selected from the group of an alkyl group, an alkoxyl group, an aryl group, and an aminocarbonyl group (-CONH₂), and may be the same or different. The same groups as described in the explanation on the formula [1] may be cited as specific examples of R₃ and R₄

As regards the synthesis via the sulfonation route, there is no particular limitation to the reaction conditions. For instance, the reaction ratio of the benzamide compound including salicylamide to the sulfonating agent, constituting the raw material, is generally 1:1 - 1:200 (molar ratio). Although a reaction solvent is not necessarily indispensable, any solvent may be used. Insofar as it is capable of dissolving the raw material benzamide compound and the sulfonating agent and, furthermore, does not get sulfonated. Examples of such solvent are dichloroethane, carbon tetrachloride, dioxane, etc. There is no particular limitation to the reaction temperature, insofar as it tends itself to an adequate reaction rate, prevents progress of side reactions which may result from abrupt heat generation and inhibits decomposition of the raw material benzamide compound and reaction product 3-sulfobenzamide. Generally, the reaction is carried out at a temperature of 0 - 150°C. By means of selecting the reaction conditions, 3-sulfobenzamide can be synthesized at a high yield.

As regards the process for producing the benzenesulfonic acid derivative compound of the invention which suits to the application as a dopant mentioned below, it is desirable that the sulfonation reaction be proceeded under such conditions that fuming sulfuric acid is selected as the sulfonating agent and is caused to contact the benzamide compound including salicylamide as the raw material by the ratio of 100 parts by weight to 500 parts by weight or less, or preferably 300 parts by weight or less, of fuming sulfuric acid. The 3-sulfobenzamide which is manufactured under the conditions may be providing a conducting polymeric material having extremely high conductivity when it is utilized for the dopant use.

There is no particular limitation to a sulfur trioxide content of the fuming sulfuric acid subject to the reaction. Although there is no particular limitation to the reaction temperature, it is preferably 100°C or lower, more preferably 80°C or lower. An adequate diluent may be used during the reaction. A solvent which does not give rise to any chemical reaction when it comes in contact with fuming sulfuric acid, such as chloroform, carbon tetrachloride, diethyl ether, may be used as the diluent. As for the reaction time, 10 min. will be long enough to produce the benzenesulfonic acid derivative compound expressed by the formula [3].

Under whatever reaction conditions should be selected, a reaction product can be deposited from the reactant solution by means of adjusting the sulfuric acid concentration in the solution after the reaction has been gone through. That is to say, after causing the benzamide compound expressed by the formula [2] to contact fuming sulfuric acid, the mixture thus prepared is converted into an aqueous solution of sulfuric acid by way of hydrolyzing excess sulfur trioxide in the fuming sulfuric acid with water or ice added thereto. The reaction product can be recovered efficiently by means of adjusting the sulfuric acid concentration of the obtained aqueous solution of sulfuric acid to 50 % by weight or more, preferably 60 - 90 % by weight. When 3-sulfobenzamide thus recovered is applied to the use as a dopant, a conducting polymeric material having a high electric conductivity may be obtained. The obtained deposit can be filtered out using a filtering devise constructed of a material noncorrodible in sulfuric acid, such as glass or "Teflon".

The filtrate, however, has a lot of aqueous sulfuric acid solution adhered to its surface. The adhered aqueous solution of sulfuric acid can be readily removed when it undergoes washing with a solvent which is capable of dissolving sulfuric acid, but does not dissolve the reaction product. Suited for such organic solvent is a ketone group compound or an ether group compound. Specific examples thereof are acetone, methyl ethyl ketone, methyl isobutyl ketone, diethyl ether, diisopropyl ether, dibutyl ether etc. There are no particular limitations to the washing condition, such as the quantity of an organic solvent to be used for washing, frequency of washing, washing temperature, etc. For instance, an organic solvent may be used by the ratio of 100 parts by weight or more of the organic solvent to 100 parts by weight of the reaction product, and washing may be carried out at a temperature of 0 - 50 °C, twice or more time.

If and when the deposit is washed with an adequate organic solvent, the amounts of alkali metal, alkaline-earth metal, or residual sulfuric acid in the reaction product may be decreased to very low levels to the extent that the benzenesulfonic acid derivative compound containing only little impurities, which are suited for the dopant use, etc. is produced.

### Dopant

A benzenesulfonic acid derivative compound expressed by a formula [4] may be utilized as a dopant to dope an electron-conjugated polymer with, and provides an electrically conducting polymeric material which exhibits electric conductivity over an extended period of time. 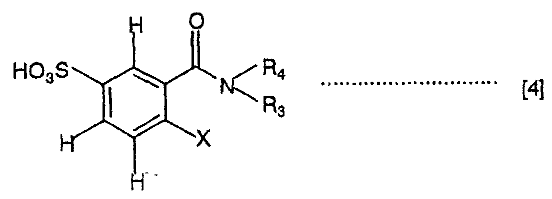 Wherein X in the formula [4] is a hydrogen or a hydroxyl group; and
R₃ and R₄ are a hydrogen or any groups selected from the group of an alkyl group, an alkoxyl group, an aryl group, and an aminocarbonyl group (-CONH₂), and may be the same or different. The same group as described in the explanation on the formula [1] may be cited as specific examples of R₃ and R₄.

There can be cited as examples of the compound which is expressed by the formula [4] and is utilizable as the dopant the following.
(1) In case X is a hydrogen, there can be exemplified as 3-sulfobenzamides such compounds as 3-sulfobenzamide, N-methyl-3-sulfobenzamide, N,N-dimethyl-3-sulfobenzamide, N-phenyl-3-sulfobenzamide, N,N-diphenyl-3-sulfobenzamide, 3-sulfobenzoylurea.
(2) In case X is a hydroxyl group, there can be exemplified as 5-sulfosalicylamides such compounds as 5-sulfosalicylamide, N-methyl-5-sulfosalicylamide, N,N-dimethyl-5-sulfosalicylamide, N-phenyl-5-sulfosalicylamide.

Any polymeric substance having a π-electron-conjugated molecular structure may be used as the electron-conjugated polymer. In particular, it is preferable to use the electron-conjugated polymer comprising at least one of a pyrrole type expressed by a following formula [5], a thiophene type expressed by a formula [6], and a aniline type expressed by a formula [7] as the repeating unit.

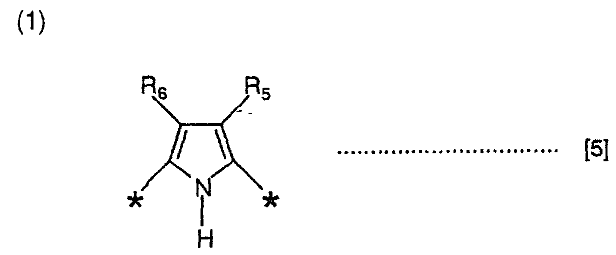

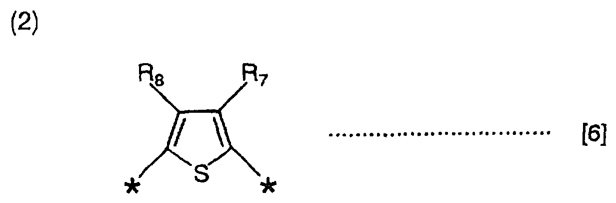

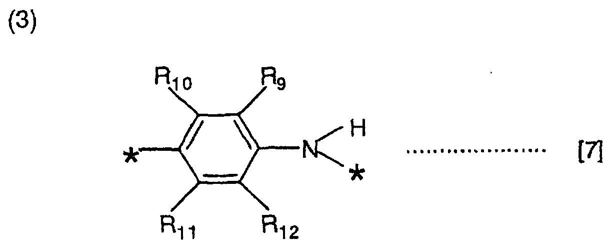

Wherein R₅ through R₁₂ in the formulae [5], [6], and [7] may be the same or different, and are a hydrogen, or a linear, branched or cyclic alkyl group or alkoxyl group having 1 - 20 carbon atoms. There can be cited as specific examples of R₅ through R₁₂ such groups as methyl group, ethyl group, propyl group, butyl group, octyl group, methoxy group, ethoxy group. The symbol " 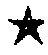 " denotes the position where the repeating unit is bonded.

Among the conjugated polymer, it is preferable to use polypyrrole, polythiophene, or polyaniline which is constituted of only one kind of repeating unit as expressed by the formula [5], [6], or [7]. In particular, it is preferable to use a polypyrrole-type conjugated polymer comprising the repeating unit expressed by the formula [5].

As for a method for doping electron-conjugated polymer with a benzenesulfonic acid derivative compound expressed by the formula [4], there may be employed any conventionally adopted method, such as the method of immersing electron-conjugated polymer in a solution of the benzenesulfonic acid derivative compound, the method of electrolytic oxidation polymerization using the benzenesulfonic acid derivative compound as a supporting electrolyte for polymerizing monomers, the method of chemical oxidation polymerization using a transition metal salt of the benzenesulfonic acid derivative compound to polymerize the monomers. In executing the doping step, conventionally well known dopants may be used insofar as such use does not go counter to the object of the invention.

In the case of the immersion doping method, any kind of solvent may be used for dissolving the benzenesulfonic acid derivative compound, so long as it is the solvent having sufficient dissolving ability such as water, acetonitrile, nitrobenzene.

In the case of the electrolytic oxidation polymerization method, doping can be accomplished by impressing specific levels of electric current or electric potential onto a solution comprising of the benzenesulfonic acid derivative compound as the supporting electrolyte and a monomer capable of constituting a repeating unit of the electron-conjugated polymer. There can be cited as examples of the monomer capable of constituting the repeating unit such compounds as pyrrole, thiophene, aniline, trans-1,2-di(2-thienyl)ethylene, and trans-1,2-di(2-thienyl) butadiene.

As for the solvent used for the polymerization reaction, any compound may be used so long as it dissolves the benzenesulfonic acid derivative compound and the monomer capable of constituting the repeating unit. Some examples are water, dimethylformamide, acetonitrile, and propylene carbonate. The electrolytic oxidation polymerization can be carried out within a temperature range of -100 - 150°C, preferably 0 - 50°C, and either one of a constant-current electrolysis method or a potentiostatic electrolysis method may be selected. As for the electrolytic oxidation polymerization, it is preferable to carry it out in an inert atmosphere, such as nitrogen and argon.

As for the doping via the chemical oxidation polymerization route, doping is executed simultaneously with the polymerization by means of causing a transition metal complex having a conjugate base of the benzenesulfonic acid derivative compound as a ligand to contact in a solvent with a monomer which is a repeating constitute unit of a polymer having the electron-conjugated molecular structure. There can be cited as examples of a center metal constituting the transition metal complex such matters as iron, cobalt, and ruthenium. Particularly preferred among them is iron. The transition metal complex is used generally by the ratio of 1 - 10 mol. to 1 mol. of the monomer.

As the monomer becoming of the repeating unit there may be used the aforesaid pyrrole, thiophene, aniline, trans-1,2-di(2-thienyl)ethylene, and trans-1,2-di(2-thienyl)butadiene. As for the solvent to be used in the reaction, any compound may be used so long as it dissolves the transition metal complex and the monomer. Some examples are water, dimethyl formamide, acetonitrile, tetrahydrofurane, and propylene carbonate, etc. The preferred polymerization temperature range is 0 -50°C and the preferred reaction time is 1 - 48 hr. It is preferable to carry out the polymerization in an inert atmosphere, such as nitrogen and argon.

As described in the foregoing, the benzenesulfonic acid derivative compound expressed by the formula [4] may be utilized as a dopant for the electron-conjugated polymer.

### Conducting Polymeric Material

A conducting polymeric material of the invention is a conducting polymeric material wherein a polymer having the electron-conjugated molecular structure comprises a benzenesulfonic acid derivative compound expressed by a formula [8] as, for example, a dopant.

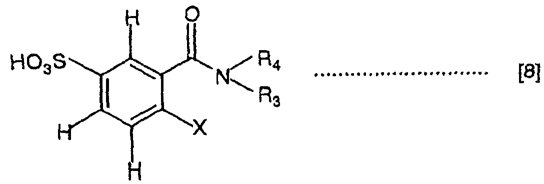

Wherein X in the formula [8] is a hydrogen or a hydroxyl group; and
R₃ and R₄ are a hydrogen or any groups selected from the group of an alkyl group, an alkoxyl group, an aryl group, and an aminocarbonyl group (-CONH₂), and may be the same or different.

As for examples of the specific compound expressed by the formula [8] and a method for including the benzenesulfonic acid derivative compound in the electron-conjugated polymer, the same compounds and methods as described in the foregoing paragraphs dealing with the dopant and the doping method may be exemplified.

According to the invention, the quantity of the benzenesulfonic acid derivative compound expressed by the formula [8] contained in the conducting polymeric material (i.e., the sum of the amount of the polymer having the electron-conjugated molecular structure and the amount of the benzenesulfonic acid derivative compound) is 5 - 95 % by weight, or preferably 30 - 70 % by weight to the conducting polymeric material.

As the polymer having electron-conjugated molecular structure any electron-conjugated polymer may be used. There can be cited as preferred examples such a π-electron-conjugated polymeric compound constituted of at least one kind of the pyrrole type expressed by the formula [5], the thiophene type expressed by the formula [6], and the aniline type expressed by the formula [7] as the repeating unit.

The polymer containing the repeating unit may be manufactured by oxidatively polymerizing the monomer capable of constituting at least one kind of repeating unit as expressed by the formula [4], [5], or [6]. There can be cited as specific examples of the monomer such compounds as pyrrole, thiophene, 3-methylthiophene, 3-octylthiophene, 3-methoxythiophene, aniline, bithiophene, terthiophene, trans-bithienylethylene, trans-bithienyl-1,4-butadiene, bipyrrole, terpyrrole, 2,5-bipyrroylthiophene, p,p'-bipyrroylbenzene, 2,5'-diphenylterpyrrole, 2,5'-dithienylbipyrrole.

In executing the manufacture of the electron-conjugated polymer, the conducting polymeric material of the invention can be obtained by an oxidation polymerization method which is carried out by causing the benzenesulfonic acid derivative compound expressed by the formula [8] to coexist in the polymerization system in which the monomer is present. The polymerization may be carried out while making another compound conventionally known as a dopant coexist in the system insofar as its presence does not go counter to the object of the invention. The oxidation polymerization may be carried out in accordance with the electrochemical oxidation polymerization method or the chemical oxidation polymerization method.

Via the electrochemical oxidation polymerization, namely, the electrolytic oxidation polymerization method, a conducting polymeric material may be obtained by first dissolving the benzenesulfonic acid derivative compound expressed by the formula [8] or its salt and the aforesaid monomer constituting the polymer in a solvent and then proceeding with polymerization of the monomer under conditions of a constant potential or a constant current. On the other hand, the chemical oxidation polymerization is carried out by causing a transition metal complex having a conjugate base of the benzenesulfonic acid derivative compound as its ligand to contact in a solvent with a monomer to be the repeating unit to constitute the polymeric compound having an electron-conjugated molecular structure. Polymerization conditions selected for either method are as shown in the detailed explanations given in the chapter dealing with the dopant.

The conducting polymeric material thus produced may be used in areas involving various electrode materials, antistatic agents, etc.

### Solid Electrolytic Capacitor

A solid electrolytic capacitor of the invention is described in detail as follows. The solid electrolytic capacitor is, in most cases, basically constructed of an anode metal which is jointed to a conductive cathode material by way of an intermediary dielectric layer. Lead-in wires are fixed onto the anode metal and the conductive cathode material, respectively.

Examples of the metal constituting the anode metal, which is generally used in the foil form, are aluminum, tantalum, etc. Its surface may be etched. On the other hand, for the conductive cathode material, an inorganic or organic electrically conductive material is used after it is fabricated into a film form. A metal cathode may be provided on its surface. Furthermore, it may be of such construction that a graphite layer is interposed between the conductive cathode material and the metal cathode for the purpose of improving the contact between the conductive cathode material and the metal cathode. The dielectric layer is generally constructed of an oxide layer produced from the anode metal. It may be produced by oxidizing the surface of the anode metal. Moreover, it can be produced by applying a liquid coating material containing an oxide of the anode metal onto the surface of the anode metal.

The solid electrolytic capacitor of the invention comprises the aforesaid conducting polymeric material as the conductive cathode material, and any conventionally used material may be used as it is for the anode metal. As for the shape of the solid electrolytic capacitor into which both the anode and the cathode are formed, any shape such as cylindrical, chip-type, may be selected.

### Examples

The invention is further described with reference to the following examples; however, it should be construed that the invention is in no way limited to those examples.

### (Example 1)

90 g of fuming sulfuric acid (with 30 % by weight of excess sulfur trioxide) was poured into a 100 mL three-neck flask, and 6.0666 g of benzamide was gently added at room temperature to the fuming sulfuric acid with stirring. The stirring was continued for 6 hr at room temperature. Thereupon, the reactant liquid was allowed to stand overnight. The reactant liquid was poured onto 100 g of ice to hydrolyze unreacted fuming sulfuric acid. Then, 1 L of pure water was added to the liquid to obtain a diluted liquid. The obtained aqueous solution was neutralized with barium carbonate, and after removing the produced barium sulfate from the solution, the solvent was removed under reduced pressure using an evaporator. To white solids thus obtained 300 mL of pure water was added, and the mixture was heated to 60°C, and insoluble matters were filtered out and the filtrate was treated with ion exchange resin. In this treatment, pH of the aqueous solution changed from 7 to 1.5. After removing the ion exchange resin and then the solvent, the resulting substance was dried under vacuum into 9.9533 g of 3-sulfobenzamide.

Identification of the obtained compound was made by FD-mass spectrum (m/z=201), ¹H-NMR, and infrared absorption spectrum. Outcomes of the analyses were as follows.

| | | |
|---|---|---|
| ¹H-NMR (D₂O) (δ ppm) | 7.63-7.69 | (1H, t, J=8 Hz) |
| | 7.95-8.00 | (2H, m) |
| | 8.20 | (1H, s) |
| Infrared absorption spectrum | Amide group | 3400 cm⁻¹, 1660cm⁻¹ |
| | Aromatic ring | 3050 cm⁻¹, 1660cm⁻¹ |

### (Examples 2 - 4)

Sulfonation reaction was carried out in accordance with the same procedure as in Example 1 using N-methylbenzamide, N,N-dimethylbenzamide, and benzoylurea as the raw material, respectively, and 3-sulfobenzamide compounds corresponding to the respective raw materials were obtained.

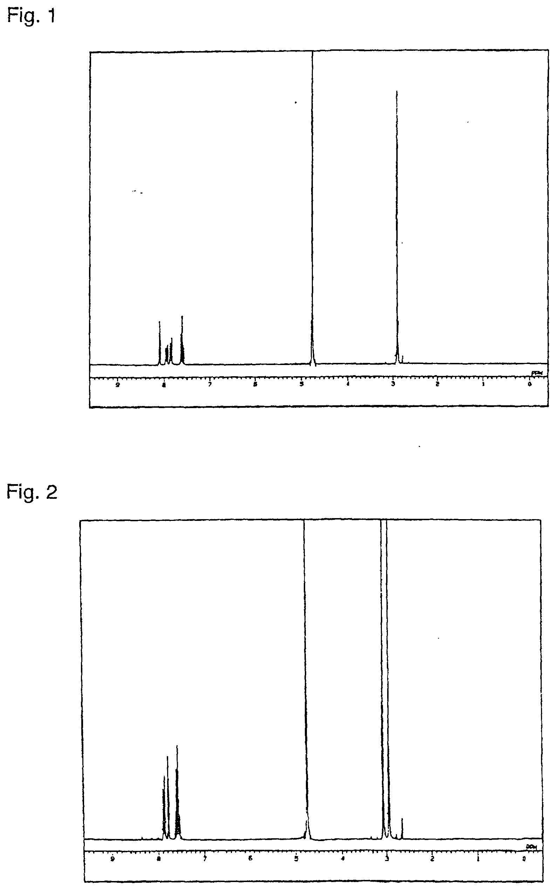

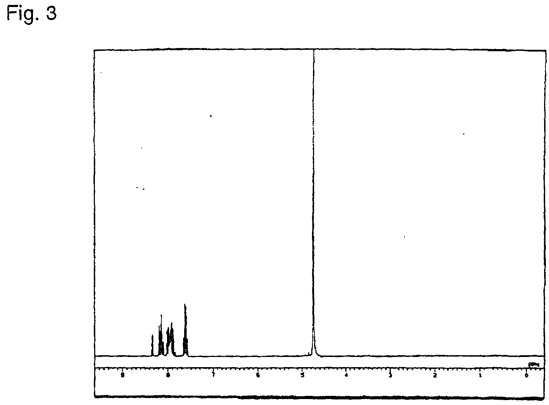

NMR charts of N-methyl-3-sulfobenzamide, N,N-dimethyl-3-sulfobenzamide, and 3-sulfobenzoyl urea were as shown in Fig. 1, Fig. 2, and Fig. 3, respectively.

### (Example 5)

2.0599 g of 3-sulfobenzamide obtained in Example 1 and 0.6729 g of pyrrole were dissolved in 100 mL of pure water, and thus was prepared a solution for electrolytic oxidation polymerization. After nitrogen displacement was carried out by way of bubbling gaseous nitrogen in the solution for about 30 min., two pieces of 2 cm square stainless steel (304) plates were immersed with a 1 cm interspace in between in the solution so as to let them function as a working electrode and a counter electrode, respectively.

With the immersed two stainless steel plates utilized as a set of electrodes, constant current (2.5 mA/cm) was passed through the circuit for 20 min., and thus was carried out electrolytic oxidation polymerization. Polyrrole film produced on the electrode was washed with pure water and then with acetone, peeled off from the electrode, and was dried under vacuum for 12 hr. The obtained film exhibited an electric conductivity of 79 S/cm as determined in accordance with the 4-probe method.

### (Examples 6 - 8)

The same procedure as in Example 5 was repeated using N-methyl-3-sulfobenzamide, N,N-dimethyl-3-sulfobenzamide, and 3-sulfobenzoylurea obtained in Examples 2 - 4, respectively, and thereby were produced pieces of polyrrole film each containing the respective 3-sulfobenzamides. Those pieces of film were tested for determining their electric conductivity. Outcomes of the tests were as shown in Table 1.

### (Comparative Examples 1 - 2)

The same procedure as in Example 5 was repeated using p-toluenesulfonic acid and 5-sulfosalicylic acid, and thereby were produced pieces of polyrrole film each containing the respective compounds. Those pieces of film were tested for determining their electric conductivity. Outcomes of the tests were as shown alongside in Table 1.

**Table 1**

| | Dopant | Electric Conductivity (S/cm) |
|---|---|---|
| Example 5 | 3-sulfobenzamide | 79 |
| Example 6 | N-methyl-3-sulfobenzamide | 105 |
| Example 7 | N,N-dimethyl-3-sulfobenzamide | 49 |
| Example 8 | 3-sulfobenzoylurea | 94 |
| Comparative Example 1 | p-toluenesulfonic acid | 93 |
| Comparative Example 2 | 5-sulfosalicylic acid | 62 |

### (Example 9)

The film obtained in Example 5 was heat treated for 8 hr in atmosphere at 150°C. After the heat treatment was gone through, the sample came to exhibit a conductivity of 78 S/cm (in accordance with the 4-probe method) which stands for 99 % in terms of the conductivity retention ratio compared with the pre-heat treatment conductivity.

### (Examples 10 - 12)

The same procedure as in Examples 9 was followed using the polypyrrole film samples obtained in Examples 6 - 8, respectively, for determining their conductivity retention ratio. Outcomes of the test were as shown in Table 2.

### (Comparative Examples 3 - 4)

The same procedure as in Examples 9 was followed using the polypyrrole film samples obtained in Comparative Examples 1-2, respectively, for determining their conductivity retention ratio. Outcomes of the test were as mentioned alongside in Table 2.

**Table 2**

| | Dopant | Electric Conductivity After Heat Treatment (S/cm) | Conductivity Retention Ratio (%) |
|---|---|---|---|
| Example 9 | 3-sulfobenzamide | 78 | 99 |
| Example 10 | N-methyl-3-sulfobenzamide | 100 | 95 |
| Example 11 | N,N-dimethyl-3-sulfobenzamide | 40 | 80 |
| Example 12 | 3-sulfobenzoylurea | 73 | 78 |
| Comparative Example 3 | p-toluenesulfonic acid | 34 | 36 |
| Comparative Example 4 | 5-sulfosalicylic acid | 32 | 51 |

### (Example 13)

90 g (50 mL) of fuming sulfuric acid (with 30 % by weight of excess sulfur trioxide) was poured into a 200 mL three-neck flask, and 20.5 g of salicylamide was gently added at room temperature to the fuming sulfuric acid with stirring. The stirring was continued for 4 hr at room temperature. Thereupon, the reactant liquid was allowed to stand overnight. The reactant liquid was poured onto 42 g of ice to hydrolyze unreacted fuming sulfuric acid. The white solids deposited therein was filtered out using a glass membrane-filter. The white solids thus filtered out was dropped into 150 mL of acetone and was washed with stirring. After filtering out the white solids, the resulting substance was dried under vacuum into 26.0 g of 5-sulfosalicylamide.

Identification of the obtained compound was made by ¹H-NMR. Outcomes of the analysis were as follows.

| | | |
|---|---|---|
| ¹H-NMR (DMSO-d6) (δ ppm) | 6.07 | (1H, broad) |
| | 6.85 | (1H, d, J=9 Hz) |
| | 7.66 | (1H, d, J=9 Hz) |
| | 7.84 | (1H, broad) |
| | 8.17 | (1H,s) |
| | 8.56 | (1H, broad) |

### (Example 14)

2.74 g of 5-sulfosalicylamide obtained in Example 13 and 1.34 g of pyrrole were dissolved in 200 mL of pure water, and thus was prepared a solution for electrolytic oxidation polymerization. After nitrogen displacement was carried out by way of bubbling gaseous nitrogen in the solution for about 30 min., two pieces of 4 cm square stainless steel (304) plates were immersed (with a 1 cm interspace) in the solution so as to let them function as a working electrode and a counter electrode, respectively.

With the immersed two stainless steel plates utilized as a set of electrodes, constant current (1.25 mA/cm) was passed through the circuit for 40 min., and thus was carried out electrolytic oxidation polymerization. Polyrrole film produced on the electrode was washed with pure water and then with acetone, peeled off from the electrode, and was dried under vacuum for 12 hr. The obtained film exhibited an electric conductivity of 114 S/cm as determined in accordance with the 4-probe method.

### (Comparative Examples 5 - 6)

The same procedure as in Example 13 was repeated using p-toluenesulfonic acid and 5-sulfosalicylic acid, and thereby were produced pieces of polyrrole film containing the respective compounds. Those pieces of film were tested for determining their electric conductivity in accordance with the 4-probe method. Outcomes of the tests were as shown alongside in Table 3.

**Table 3**

| | Dopant | Electric Conductivity (S/cm) |
|---|---|---|
| Example 13 | 5-sulfosalicylamide | 114 |
| Comparative Example 5 | p-toluenesulfonic acid | 93 |
| Comparative Example 6 | 5-sulfosalicylic acid | 99 |

### (Example 15)

The film obtained in Example 14 was heat treated for 8 hr in atmosphere at 150°C. After the heat treatment was gone through, the sample exhibited a conductivity of 109 S/cm (in accordance with the 4-probe method) which stands for 96 % in terms of the conductivity retention ratio compared with the pre-heat treatment conductivity. Outcomes of the test were as shown in Table 4.

### (Comparative Examples 7 - 8)

The same procedure as in Examples 15 was followed using the polypyrrole film samples obtained in Comparative Examples 5-6, respectively, for determining their conductivity retention ratio. Outcomes of the test were as shown alongside in Table 4.

**Table 4**

| | Dopant | Electric Conductivity After Heat Treatment (S/cm) | Conductivity Retention Ratio (%) |
|---|---|---|---|
| Example 14 | 5-sulfosalicylamide | 109 | 96 |
| Comparative Example 7 | p-toluenesulfonic acid | 34 | 36 |
| Comparative Example 8 | 5-sulfosalicylic acid | 94 | 95 |

### (Example 16)

360 g of fuming sulfuric acid (with 30 % by weight of excess sulfur trioxide) was poured into a 500 mL three-neck flask, and 121 g of benzamide was gently added at room temperature to the fuming sulfuric acid with stirring. The stirring was continued for 1 hr at 70 °C. Thereupon, the reactant was allowed to stand overnight. The reactant liquid was poured onto 100 g of ice to hydrolyze unreacted sulfur trioxide. Thereupon, 125 g of ice was added to the liquid for depositing a reaction product. The deposited reaction product was filtered out using a glass membrane-filter and washed with acetone. The resulting substance was dried under vacuum for 12 hr into 53 g of 3-sulfobenzamide.

Non-existence of any heavy metal content in the obtained 3-sulfobenzamide was confirmed by elementary analysis. Also, it was confirmed by ion chromatogram that the sulfuric acid radical was 1 % or less.

### (Examples 17 - 18)

Sulfonation reaction was carried out in accordance with the same procedure as in Example 16 using benzoylurea and salicylamide as the raw materials and thus were obtained 3-sulfobenzamide compounds corresponding to the respective raw materials.

### (Example 19)

90 g of fuming sulfuric acid (with 30 % by weight of excess sulfur trioxide) was poured into a 200 mL three-neck flask, and 22.5 g of N,N-dimethylbenzamide was gently added at room temperature to the fuming sulfuric acid with stirring. The stirring was continued for 2 hr at 70°C. Thereupon, the reactant liquid was allowed to stand so as to cool down to room temperature. The reactant liquid was poured onto 40 g of ice and neutralized with barium carbonate. After removing the salt produced in the neutralization step, hydrolysis was caused to take place by adding 5 mL of concentrated sulfuric acid. Then, after removing the salt produced in the hydrolysis step, the solvent was removed under reduced pressure. The obtained solids was washed with acetone and dried under vacuum at room temperature into 26.1 g of 3-sulfo-N,N-dimethylbenzamide.

### (Example 20)

4.0 g of 3-sulfobenzamide obtained in Example 16 and 1.3 g of pyrrole were dissolved in 200 mL of pure water, and thus was prepared a solution for electrolytic oxidation polymerization. After nitrogen displacement was carried out by way of bubbling gaseous nitrogen in the solution for about 10 min., two pieces of 2 cm square stainless steel (304) plates were immersed (with a 1 cm interspace) in the solution so as to let them function as a working electrode and a counter electrode, respectively.

With the immersed two stainless steel plates utilized as a set of electrodes, constant current (2.5 mA/cm) was passed through the circuit for 20 min., and thus was carried out electrolytic oxidation polymerization. Polyrrole film produced on the electrode was washed with pure water and then with acetone, peeled off from the electrode, and was dried under vacuum for 12 hr. The obtained film exhibited an electric conductivity of 165 S/cm as determined in accordance with the 4-probe method.

### (Examples 21 - 22)

Tests were conducted in the same manner as in Example 20 using 3-sulfobenzoylurea and 5-sulfosalicylamide obtained in Examples 17 - 18, respectively, for determining their electric conductivity.

### (Example 23)

4.6 g of N,N-dimethyl-3-sulfobenzamide obtained in Example 19 and 1.3 g of pyrrole were dissolved in 200 mL of pure water, and thus was prepared a solution for electrolytic oxidation polymerization. After nitrogen displacement was carried out by way of bubbling gaseous nitrogen in the solution for about 10 min., two pieces of 2 cm square stainless steel (304) plates were immersed (with a 1 cm interspace) in the solution so as to let them function as a working electrode and a counter electrode, respectively.

With the immersed two stainless steel plates utilized as a set of electrodes, constant current (2.5 mA/cm) was passed through the circuit for 20 min., and thus was carried out electrolytic oxidation polymerization. Polyrrole film produced on the electrode was washed with pure water and then with acetone, peeled off from the electrode, and was dried under vacuum for 12 hr. The obtained film samples were tested for determining their electric conductivity in accordance with the 4-probe method. Outcomes of the tests were as shown in Table 5.

**Table 5**

| | Dopant | Electric Conductivity (S/cm) |
|---|---|---|
| Example 20 | 3-sulfobenzamide | 165 |
| Example 21 | 3-sulfobenzoylurea | 128 |
| Example 22 | 5-sulfosalicylamide | 114 |
| Example 23 | N,N-dimethyl-3-sulfobenzamide | 55 |

### (Examples 24 - 27)

The films samples obtained in Examples 20-23, respectively, were heat treated for 8 hr in atmosphere at 150°C. After the heat treatment was gone through, the samples were tested for determining their conductivity. Outcomes of the test and the conductivity retention ratio, which was the compared value of the post-heat treatment conductivity with the pre-heat treatment conductivity, were as shown in Table 6.

**Table 6**

| | Dopant | Electric Conductivity After HeatTreatment (S/cm) | Conductivity Retention Ratio (%) |
|---|---|---|---|
| Example 24 | 3-sulfobenzamide | 162 | 92 |
| Example 25 | 3-sulfobenzoylurea | 105 | 83 |
| Example 26 | 5-sulfosalicylamide | 109 | 96 |
| Example 27 | N,N-dimethyl-3-sulfobenzamide | 31 | 57 |

### Industrial Applicability of the Invention:

The benzenesulfonic acid derivative compound of the invention is utilizable as a dopant for an electron-conjugated polymers. The conducting polymeric material obtained after the doping step has been gone through possesses high conductivity and excellent heat resistance. It exhibited a high conductivity retention ratio even after heat treatment was gone through. Hence, the conducting polymeric material thereby obtained may be utilizable as raw material for capacitor electrode, battery electrodes, etc.

Furthermore, this conducting polymeric material may be used as a conducting material for a solid electrolytic capacitor cathode. It exhibits small impedance at resonance frequency and good high frequency characteristics, and hence provides a solid electrolytic capacitor which is free from serious decline in performance characteristics at high temperatures.

## Claims

1. A benzenesulfonic acid derivative compound expressed by a formula [1], 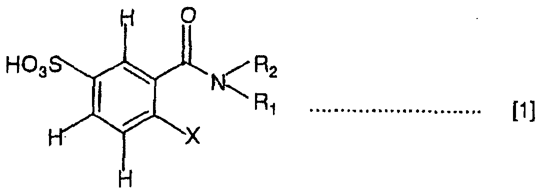 wherein X is a hydrogen or a hydroxyl group; and
R₁ and R₂ are a hydrogen or any groups selected from the group of an alkyl group, an alkoxyl group, an aryl group, and an aminocarbonyl group, provided that they may be the same or different except that R₁ and R₂ are a hydrogen; and in case either one of R₁ and R₂ is a hydrogen, the other is a group selected from the group of an alkyl group, an alkoxyl group, an aryl group and an aminocarbonyl group.

2. A benzenesulfonic acid derivative compound as claimed in claim 1, wherein R₁ and R₂ in the formula [1] are each either one of a hydrogen, a methyl group, a phenyl group and an aminocarbonyl group.

3. A benzenesulfonic acid derivative compound as claimed in claim 1, wherein the compound expressed by the formula [1] is either one of N-methyl-3-sulfobenzamide, N,N-dimethyl-3-sulfobenzamide, N-phenyl-3-sulfobenzamide, N,N-diphenyl-3-sulfobenzamide, and 3-sulfobenzoylurea.

4. A benzenesulfonic acid derivative compound as claimed in claim 1, wherein the compound expressed by the formula [1] is either one of N-methyl-5-sulfosalicylamide, N,N-dimethyl-5-sulfosalicylamide, and N-phenyl-5-sulfosalicylamide.

5. A process for producing a benzenesulfonic acid derivative compound comprising manufacturing a sulfonate expressed by a formula [3] by way of reacting 100 parts by weight of a benzamide compound expressed by a formula [2] with 500 parts by weight or less of a fuming sulfuric acid, 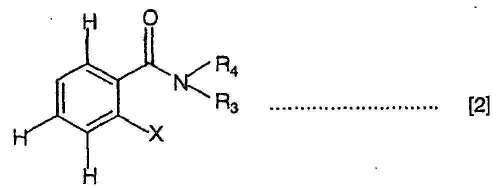 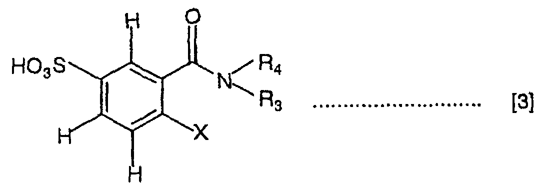 wherein X is a hydrogen or a hydroxyl group; and
R₃ and R₄ are a hydrogen or any groups selected from the group of an alkyl group, an alkoxyl group, an aryl group, and an aminocarbonyl group, and may be the same or different.

6. A process for producing a benzenesulfonic acid derivative compound comprising:
reacting a benzamide compound expressed by a formula [2] with a fuming sulfuric acid,
adjusting a sulfuric acid concentration in a reactant solution to 50 % weight or
more, and
depositing a sulfonate expressed by a formula [3] from the reactant solution with acid, 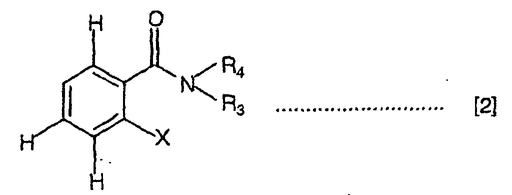 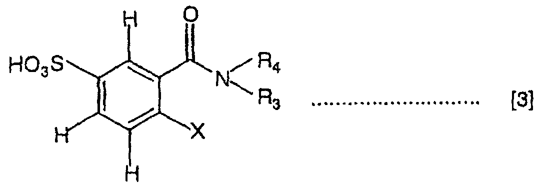 wherein X is a hydrogen or a hydroxyl group; and
R₃ and R₄ are a hydrogen or any groups selected from the
group of an alkyl group, an alkoxyl group, an aryl group, and an aminocarbonyl group, and may be the same or different.

7. A process for producing a benzenesulfonic acid derivative compound, further comprising washing a deposit produced from the reactant solution as claimed in claim 5 or claim 6, with an organic solvent.

8. A process for producing a benzenesulfonic acid derivative compound as claimed in claim 7, wherein the organic solvent is any one of ketone or ether compound.

9. A process for producing a benzenesulfonic acid derivative compound as claimed in claim 7, wherein the organic solvent is at least one kind of compound selected from the group of methyl ethyl ketone, methyl isobutyl ketone, diethyl ether, diisopropyl ether, and dibutyl ether.

10. A dopant comprising of a benzenesulfonic acid derivative compound expressed by a formula [4], 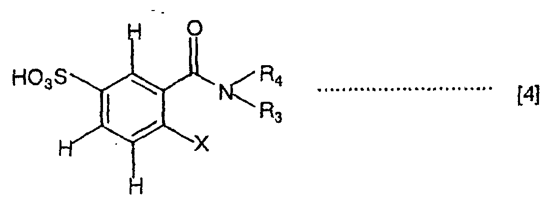 wherein X is a hydrogen or a hydroxyl group; and
R₃ and R₄ are a hydrogen or any groups selected from the group of an alkyl group, an alkoxyl group, an aryl group, and an aminocarbonyl group, and may be the same or different.

11. A dopant as claimed in claim 10, wherein R₃ and R₄ in the formula [4] are each either one of a hydrogen, a methyl group, a phenyl group, and an aminocarbonyl group.

12. A dopant as claimed in claim 10, wherein the compound expressed by the formula [4] is either one of 3-sulfobenzamide, N-methyl-3-sulfobenzamide, N,N-dimethyl-3-sulfobenzamide, N-phenyl-3-sulfobenzamide, N,N-diphenyl-3-sulfobenzamide, and 3-sulfobenzoylurea.

13. A dopant as claimed in claim 10, wherein the compound expressed by the formula [4] is either one of 5-sulfosalicylamide, N-methyl-5-sulfosalicylamide, N,N-dimethyl-5-sulfosalicylamide, and N-ethyl-5-sulfosalicylamide.

14. A dopant as claimed in claim 10, wherein the compound expressed by the formula [4] is either 3-sulfobenzamide or 5-sulfosalicylamide.

15. A conducting polymeric material, wherein a polymer having the electron-conjugated molecular structure comprises a benzenesulfonic acid derivative compound expressed by a formula [8], 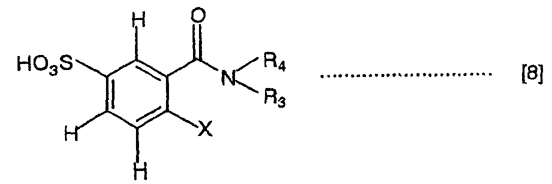 wherein X is a hydrogen or a hydroxyl group; and
R₃ and R₄ are a hydrogen or any groups selected from the group of an alkyl group, an alkoxyl group, an aryl group, and an aminocarbonyl group, and may be the same or different.

16. A conducting polymeric material as claimed in claim 15, wherein the polymer comprises a π- electron-conjugated polymeric compound having at least one kind of repeating unit expressed by a formula [5], [6] and [7], 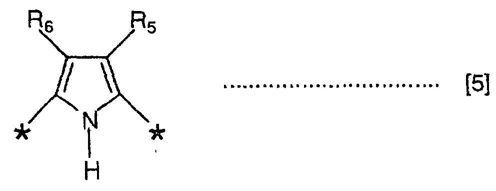 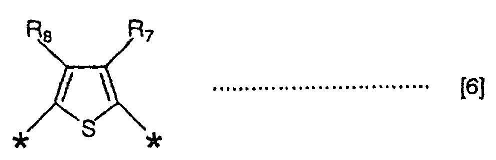 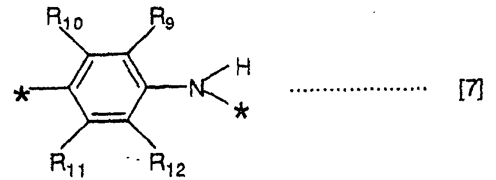 wherein R₅ through R₁₂ may be the same or different, and are each either one of a hydrogen, an alkyl group, or an alkoxyl group, and the symbol " 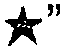 " denotes the position where the repeating unit is bonded.

17. A conducting polymeric material as claimed in claim 15, wherein the polymer is polypyrrole.

18. A solid electrolytic capacitor comprising a conducting polymeric material as claimed in either one of claims 15-17 which is included as a conducting material for a cathode.
